# EUROPÄISCHE PATENTSCHRIFT

(11) **EP 3 200 849 B1**
(45) Veröffentlichungstag und Bekanntmachung des Hinweises auf die Patenterteilung: **12.09.2018**
(21) Anmeldenummer: 15778247.5
(22) Anmeldetag: 01.10.2015
(51) Int. Cl.: A61M 1/36

(54) **VORRICHTUNG MIT EINEM BEUTELFÖRMIGEN BEHÄLTNIS SOWIE VERFAHREN ZUM GASBLASENFREIEN BEFÜLLEN EINES PUMPENBETRIEBENEN, HOHLLEITUNGSGESTÜTZTEN FLÜSSIGKEITSKREISLAUFES MITTELS DER VORRICHTUNG**
DEVICE WITH A BAG-SHAPED CONTAINER, AND METHOD FOR FILLING A PUMP-OPERATED HOLLOW LINE-SUPPORTED LIQUID CIRCUIT WITHOUT FORMING GAS BUBBLES USING THE DEVICE
DISPOSITIF MUNI D'UN RÉCIPIENT EN FORME DE SAC, AINSI QUE PROCÉDÉ PERMETTANT DE REMPLIR SANS BULLES DE GAZ, AU MOYEN DU DISPOSITIF, UN CIRCUIT DE LIQUIDE BASÉ SUR UNE CONDUITE CREUSE ET ENTRAÎNÉ PAR UNE POMPE

(30) Priorität: 02.10.2014 DE 102014014725
(43) Veröffentlichungstag der Anmeldung: 09.08.2017
(73) Patentinhaber: ResuSciTec GmbH, 79108 Freiburg (DE)
(72) Erfinder: BENK, Christoph, 79106 Freiburg (DE); GRUDKE, Jürgen, 47839 Krefeld (DE); LOUOBA, Joel, 79424 Auggen (DE)
(74) Vertreter: Rösler, Uwe
(86) Internationale Anmeldenummer: PCT/EP2015/072720
(87) Internationale Veröffentlichungsnummer: WO 2016/050924

(56) Entgegenhaltungen:
- DE-A1- 3 347 183
- DE-A1- 19 750 062
- DE-U1- 20 213 693
- US-A- 4 734 269

## Beschreibung

### Technisches Gebiet

Die Erfindung bezieht sich auf eine Vorrichtung mit einem beutelförmigen Behältnis, dessen Beutelwand zumindest in einem Bereich flexibel ausgebildet ist, in dem zwei Hohlkanalabschnitte, ein erster und ein zweiter Hohlkanalabschnitt, die Beutelwand fluiddicht durchragen. Beide Hohlkanalabschnitte weisen jeweils ein offenes Kanalende innerhalb des Beutels auf, die zum lösbaren fluiddichten Konnektieren miteinander ausgebildet sind. Die Vorrichtung dient insbesondere dem gasblasenfreien Befüllen eines pumpenbetriebenen, hohlleitungsgestützten Flüssigkeitskreislaufes mit einer Flüssigkeit. In diesem Zusammenhang wird ein Verfahren beschrieben, mit dem ein pumpenbetriebener, hohlleitungsgestützter Flüssigkeitskreislauf, in dem vorzugsweise ein Oxygenator integriert ist, unter Verwendung der Vorrichtung gasblasenfrei mit einer Flüssigkeit befüllt wird.

### Stand der Technik

Aus der Druckschrift DE 33 47 183 A1 sind ein Verfahren sowie eine Vorrichtung zur blasenfreien Verbindung von Füllschläuchen, insbesondere für Blut oder dergleichen zu entnehmen. Hierbei kommt ein flexibel ausgebildeter, fluiddichter Beutel zum Einsatz, durch dessen Beutelwand zwei Schlauchenden eines Hohlleitungssystems fluiddicht hindurchragen und im Inneren des Beutels offen münden. Das Befüllen des Hohlleitungssystems erfolgt über den Beutel, wobei während der Befüllung der Beutel und die in den Beutel hineinragenden Schlauchenden die höchste Position relativ zum Schwerkraftsvektor im Hohlleitungssystem einnehmen. Auf diese Weise können mögliche in der Flüssigkeit eingeschlossene und somit mitgeführte Gasblasen, insbesondere in Form von Luftblasen, auftriebsbedingt über die Schlauchleitungsenden innerhalb des Beutels entweichen. Nach einer gewissen Verweildauer für den auftriebsgetriebenen Entgasungsvorgang werden beide Schlauchenden innerhalb des mit Flüssigkeit befüllten Beutelvolumens fluiddicht konnektiert. Dabei wird sichergestellt, dass beide Schlauchleitungsenden während des Konnektierens keinen Luftkontakt haben. Die flexible Beutelwand ist hierzu lichttransparent ausgebildet, so dass der Verbindungsvorgang manuell und visuell überwacht durchgeführt werden kann.

Aus der Druckschrift US 4,734,269 geht eine Beutelvorrichtung zur Gasabscheidung für einen innerhalb eines hohlleitungsgestützten Flüssigkeitskreislaufes geführten Flüssigkeitsstromes hervor. Der Beutel verfügt über ein in sich geschlossenes Beutelvolumen, in das eine Zuleitung sowie beabstandet zu dieser eine Ableitung einmünden. Die Leitungsöffnungen der Zu- und Ableitung sind jeweils bündig zur Beutelinnenwand ausgebildet. Innerhalb des Beutelvolumens ist zudem ein Filterelement fest eingebracht, dessen Filterwand ein Filtervolumen teilweise umfasst und im Bereich der Zuleitung gegenüberliegend taschenförmig geöffnet ausgebildet ist, so dass ein durch die Zuleitung in den Beutel einströmender Flüssigkeitsstrom in das Filtervolumen gerichtet ist. Die Filterwand separiert beim strömungsbedingten Durchtritt der Flüssigkeit durch die Filterwand in das übrige Beutelvolumen mögliche Gasanteile, die über eine obere, in den Beutel und in das Filtervolumen ragende Entgasungshohlleitung entweichen kann. Die nach Durchtritt durch die Filterwirkung der Filterwand entgaste Flüssigkeit rezirkuliert über die offen in das Beutelvolumen mündende Ableitung.

In einer weiteren Druckschrift, der US 5,573,526 ist ein Reservoirbeutel für eine Flüssigkeit beschrieben, vorzugsweise zur Bevorratung von Blutreserven zu deren schnellen und sicheren fluidischen Anbindung an extrakorporale bzw. kardiopulmonale Kreislaufsysteme. In ähnlicher Weise wie beim vorstehend erläuterten Beutelsystem verfügt der bekannte Reservoirbeutel innerhalb des Beutelvolumens über einen Filtereinsatz, in dessen von einer Filterwand umschlossenen Filtervolumen eine durch die Beutelwand fluiddicht hindurchragende Zuleitung einmündet. Der Filtereinsatz schließt ein gegenüber dem übrigen Beutelvolumen abgeschlossenes Filtervolumen ein, in dessen oberen Volumenbereich eine Entgasungsleitung einmündet, die den Beutel fluiddicht durchragt. Das Filtervolumen ist gegenüber dem restlichen Beutelvolumen über die Filterwand begrenzt, so dass aufgrund der Filterwirkung Flüssigkeit in das übrige Beutelvolumen blasenfrei gelangen kann, aus dem die Flüssigkeit über die dort einmündende Ableitung bspw. einem extrakorporalen Kreislaufsystem zugeführt werden kann.

Der Druckschrift US 5,935,093 ist ein Kardiotomiebehälter zu entnehmen, dessen Ausgestaltung unter der Maßgabe optimiert ist die Menge an Primingflüssigkeit, die für einen gasblasenfreien Fluidanschluss an den Oxiginator einer Herz-Lungen-Maschine erforderlich ist, möglichst gering zu halten, um negative Auswirkungen auf den mit der Herz-Lungen-Maschine verbundenen Patienten zu minimieren. Der bekannte Kardiotomiebehälter weist drei Volumina auf, nämlich ein Mischvolumen, ein Speichervolumen sowie ein Kardiotomievolumen. Über einen Kardiotomieeinlassport wird das mit einer Filter-/Entschäumungsanordnung versehene Kardiotomievolumen mit Kardiotomieblut gefüllt, das im Anschluss das Speichervolumen füllt. Über am Mischvolumen angebrachte Einlassports gelangt venöses Blut des Patienten in das Mischvolumen, in dem eine Durchmischung mit dem Kardiotomieblut aus dem Speichervolumen erfolgt. Letztlich gelangt das Blutgemisch über einen am Mischvolumen angebrachten Auslassport in den Patienten. Der Kardiotomiebehälter dient als Volumenzwischenpuffer für den Betrieb einer Herz-Lungen-Maschine, bei der mögliche Blutflussunterschiede im extrakorporalen Blutkreislauf kompensiert werden können.

### Darstellung der Erfindung

Der Erfindung liegt die Aufgabe zugrunde, eine Vorrichtung gemäß vorstehend genannter Druckschrift, DE 33 47 183 A1, mit einem beutelförmigen Behältnis mit einer Beutelwand, die zumindest in einem Bereich flexibel ausgebildet ist, in dem zwei Hohlkanalabschnitte die Beutelwand fluiddicht durchragen und jeweils ein offenes Kanalende innerhalb des Beutels aufweisen, die zum lösbaren, fluiddichten Konnektieren miteinander ausgebildet sind, derart weiterzubilden, dass ein schnelles, und gasblasenfreies Befüllen eines pumpenbetriebenen, hohlleitungsgestützten Flüssigkeitskreislaufes ermöglicht wird.

Gilt es zum Teil großvolumige pumpengetriebene, hohlleitungsgestützte Flüssigkeitskreisläufe in möglichst kurzer Zeit gasblasenfrei zu Befüllen, so bedarf es großer Fördergeschwindigkeiten, mit denen eine Flüssigkeit durch den hohlleitungsgestützten Flüssigkeitskreislauf hindurchgepumpt wird. Dabei treten Strömungsgeschwindigkeiten von fünf bis zehn Liter pro Minute auf, die jedoch bei Verwendung bisheriger Befüllungsmethoden zu erheblichen Probleme führen sowohl bei der Entgasung als auch bei einem anschließenden gasblasenfreien Konnektieren zweier flüssigkeitsbefüllter Schlauchenden. Es soll daher eine Möglichkeit geschaffen werden, mit der ein schnelles Befüllen eines pumpengetriebenen, hohlleitungsgestützten Flüssigkeitskreislaufes ermöglicht wird, wobei sichergestellt sein soll, dass das Konnektieren beider innerhalb des Beutels befindlichen, offenen Kanalenden gasblasenfrei durchgeführt werden kann.

Die Lösung der der Erfindung zugrunde liegenden Aufgabe ist im Anspruch 1 angegeben. Gegenstand des Anspruches 9 ist eine bevorzugte Verwendung der lösungsgemäß ausgebildeten Vorrichtung. Im Anspruch 11 ist ein lösungsgemäßes Verfahren zum gasblasenfreien Befüllen eines pumpenbetriebenen, hohlleitungsgestützten Flüssigkeitskreislaufes mit einer Flüssigkeit angegeben. Den Erfindungsgedanken in vorteilhafter Weise weiterbildende Merkmale sind Gegenstand der Unteransprüche sowie der weiteren Beschreibung insbesondere unter Bezugnahme auf das illustrierte bevorzugte Ausführungsbeispiel beschrieben.

Eine lösungsgemäße Vorrichtung mit einem beutelförmigen Behältnis, im Weiteren kurz Beutel genannt, gemäß den Merkmalen des Oberbegriffes des Anspruches 1, zeichnen sich derart aus, dass innerhalb des Beutels ein erster Filtereinsatz eingebracht ist, der innerhalb eines von dem Beutel zumindest teilweise umfassten Volumens, dem sogenannten Beutelvolumen, ein erstes inneres Filtervolumen mit einer ersten Filterwand zumindest teilweise umfasst. Durch die erste Filterwand ragt ein mit dieser lokal verbundener, schlauchförmiger Fluidleitungsabschnitt, der beidseitig offen ausgebildet ist und einseitig mittel- oder unmittelbar in das erste Filtervolumen und andererseits unmittelbar in das Beutelvolumen mündet. Zudem ist das innerhalb des Beutels befindliche Kanalende des ersten Hohlkanalabschnittes zum lösbar fluiddichten Konnektieren mit dem offen in das Beutelvolumen mündenden Fluidleitungsabschnitt ausgebildet.

Der lösungsgemäß ausgebildete Beutel unterscheidet sich von der bekannten Vorrichtung zum luftblasenfreien Verbinden zweier Füllschläuche gemäß der vorstehend gewürdigten Druckschrift DE 33 47 183 A1 zumindest sowohl durch das Vorsehen des ersten Filtereinsatzes innerhalb des Beutelvolumens sowie durch den mit der ersten Filterwand verbundenen, schlauchförmigen Fluidleitungsabschnitt, der eine fluidische Verbindung zwischen dem ersten inneren Filtervolumen und dem übrigen Beutelvolumen ermöglicht. Neben der fluidischen Verbindung zwischen beiden Volumina dient jedoch der schlauchförmige Fluidleitungsabschnitt insbesondere als Verbindungsstück zum lösbar fluiddichten Konnektieren des in den Beutel einmündenden ersten Hohlkanalabschnittes, so dass das Kanalende des ersten Hohlkanalabschnitt bedarfsweise entweder über den schlauchförmigen Fluidleitungsabschnitt fluiddicht mit dem ersten Filtervolumen sowie alternativ auch mit dem offenen Kanalende des innerhalb des Beutels mündenden zweiten Hohlkanalabschnitt verbindbar ist. Hierdurch vereint die lösungsgemäße Vorrichtung zwei unterschiedliche Funktionen, nämlich zum einen die Funktion der Gasabscheidung eines mit Gasblasen versetzten Flüssigkeitsstromes, der mit hoher Strömungsgeschwindigkeit die lösungsgemäße Beutelvorrichtung durchströmt, und zum anderen die Funktion des gasblasenfreien fluiddichten Konnektierens zweier flüssigkeitsgefüllter Hohlkanalabschnitte.

In vorteilhafter Weise ist der schlauchförmige Fluidleitungsabschnitt lokal fest mit dem ersten Filtereinsatz, aber ansonsten freitragend verbunden, wobei die erste Filterwand eine dem schlauchförmigen Fluidleitungsabschnitt zugeordnete Außenwand peripher fluiddicht umfasst. Somit besteht an diesem Verbindungsbereich zwischen dem Fluidleitungsabschnitt und der ersten Filterwand keine Öffnung in der Filterwand, durch die ein ungefilteter Flüssigkeitsaustausch zwischen dem den ersten Filtereinsatz umgebenden Beutelvolumen und dem ersten Filtervolumen stattfinden kann.

Zum fluiddichten Konnektieren des innerhalb des Beutels befindlichen Kanalendes des ersten Hohlkanalabschnittes mit dem offen in das Beutelvolumen mündenden Fluidleitungsabschnitt ist vorzugsweise der erste Hohlkanalabschnitt aus einem steiferen Material gefertigt als das Material, aus dem der schlauchförmige Fluidleitungsabschnitt besteht. Vorzugsweise besteht der schlauchförmige Fluidleitungsabschnitt aus einem biokompatiblen PVC-Material, wohingegen der erste Hohlkanalabschnitt aus Polycarbonat gefertigt ist. Durch die unterschiedlichen Elastizitäten kann der flexiblere, schlauchförmige Fluidleitungsabschnitt fluiddicht auf das innerhalb des Beutels befindliche Kanalende des ersten Hohlkanalabschnittes aufgeschoben werden. Selbstverständlich ist es alternativ möglich, die vorstehende Materialwahl in umgekehrter Weise vorzunehmen.

Alternativ ist es auch denkbar die offen in das Beutelvolumen mündenden Kanalenden des ersten Hohlkanalabschnittes und des schlauchförmigen Fluidleitungsabschnittes mittels eines Fluidkonnektors fluiddicht zu verbinden. Hierbei weist der Konnektor beidseitig sich konisch verjüngende Konnektorenden auf, die unter Ausbildung einer fluiddichten Klemm- und Reibschlussverbindung jeweils in die offenen Kanalenden fügbar sind. Ein derartiger Fluidkonnektor kann auch einseitig unlösbar fest mit einem der beiden Kanalenden verbunden sein.

Die Beutelwand ist zumindest im Bereich der die Beutelwand durchragenden ersten und zweiten Hohlkanalabschnitte flexibel ausgebildet und besteht darüber hinaus aus einem lichttransparenten Material, so dass die Handhabung des Beutels und insbesondere das Verbinden und Lösen der im Beutel befindlichen Kanalenden beider Hohlkanalabschnitte sowie des schlauchförmigen Fluidleitungsabschnittes unter Inaugenscheinnahme manuell über die Beutelwand von außen durchgeführt werden können.

Auch zum fluiddichten Verbinden bzw. Konnektieren der beiden innerhalb des Beutels befindlichen offenen Kanalenden des ersten und zweiten Hohlkanalabschnittes verfügen diese über unterschiedliche Materialelastizitäten, so dass ein fluiddichtes gegenseitiges Ineinanderfügen der offenen Kanalenden beider Hohlkanalabschnitte zum Zwecke eines gasblasenfreien Konnektierens möglich ist.

Der vorzugsweise fest innerhalb des Beutelvolumens fixierte, erste Filtereinsatz besteht aus einem Netzfilter, einem sogenannten Screening-Filter, mit einer Filterwand mit Filterporengrößen vorzugsweise zwischen 20 und 200 µm, insbesondere 40, 80 oder 120 µm. Die Filterwand kann das erste innere Filtervolumen vollständig umschließen, ebenso ist es möglich den ersten Filtereinsatz beutel- oder sackartig auszubilden, d. h. die Filterwand verfügt in diesem Fall über eine einseitige, vorzugsweise obere Öffnung, die dem unteren Filterbeutelboden gegenüberliegt, an dem der die Filterwand durchragende Fluidleitungsabschnitt angebracht ist.

Zur Verbesserung der Filterwirkung, d. h. der mit dem Filtereinsatz erzielbaren Gasabscheidung von einem in das Filtervolumen einströmenden Flüssigkeitsstroms, hat es sich insbesondere bei hohen Strömungsgeschwindigkeiten als vorteilhaft erwiesen, wenigstens einen zweiten Filtereinsatz innerhalb des ersten Filtervolumens einzubringen, wobei der zweite Filtereinsatz ein zweites Filtervolumen mit einer zweiten Filterwand zumindest teilweise umschließt, in das der in das erste Filtervolumen mündende Fluidleitungsabschnitt mündet. Gleichsam der ersten Filterwand grenzt auch die zweite Filterwand fluiddicht lokal an dem Fluidleitungsabschnitt an.

Wie den weiteren Ausführungen zur Filterwirkung entnommen werden können, dient der zweite Filtereinsatz zum einen für eine zweistufige Gasphasenabscheidung, bei der eine in das zweite Filtervolumen einströmende Flüssigkeitsströmung bei Durchtritt durch die zweite Filterwand eine erste und bei nachfolgendem Durchtritt durch die erste Filterwand eine zweite Filterung erfährt, bei der jeweils Gasanteile zurückgehalten bzw. separiert werden, zum anderen vermag der zweite Filtereinsatz den Strömungsdruck, der ansonsten unvermindert auf die erste Filterwand lasten würde, zu reduzieren. Hierdurch kann überdies Einfluss auf die Flüssigkeitsströmung innerhalb des ersten Filtervolumens genommen werden. Die zweistufige Filterwirkung, die bei Bedarf auch durch weitere Filtereinsätze optimiert werden kann, zeigt sich insbesondere bei Fluidströmungsgeschwindigkeiten von 5 Liter pro Minute und mehr als besonders wirksam.

Zu Zwecken der Abführung der innerhalb des wenigstens ersten Filtervolumens separierten Gasanteils mündet wenigstens eine Entgasungsleitung in das erste Filtervolumen, über die Gasanateile abgeführt werden können. Optional kann eine zweite Entgasungsleitung vorgesehen sein, die durch den ersten und zweiten Filtereinsatz hindurch in das zweite Filtervolumen mündet, über die sich dort ansammelnde Gasanteile abgeführt werden können.

Gilt es den Beutel mit einer Flüssigkeit zu Befüllen, die im Weiteren der Befüllung eines pumpenangetriebenen, hohlleitungsgestützten Flüssigkeitskreislaufes dient, so ist wenigstens eine Füllleitung vorgesehen, die in das erste Filtervolumen mündet. Von Seiten des ersten Filtervolumens gelangt die zugeführte Flüssigkeit nach Durchtritt durch die erste Filterwand in das umliegende Beutelvolumen, aus dem die Flüssigkeit im Weiteren durch den zweiten Hohlkanalabschnitt einem externen Flüssigkeitskreislauf zugeführt wird. Auch können weitere, vorzugsweise in das erste Filtervolumen mündende Fluidleitungen, je nach Anwendungsfall vorgesehen werden.

In vorteilhafter Weise münden die vorgenannten, als Hohlleitungen ausgebildeten Entgasung-, Füll- sowie Fluidleitungen in einem Bereich des ersten Filtervolumens, der dem Fluidleitungsabschnitt diametral gegenüberliegt. In allen praktischen Anwendungen wird der Beutel relativ zum Schwerkraftsvektor stets vertikal orientiert, so dass die vorstehend genannten Hohlleitungen allesamt oberhalb des Fluidleitungsabschnittes in das erste Filtervolumen münden. Weitere Einzelheiten zur körperlichen Ausgestaltung der lösungsgemäßen Beutelvorrichtung sind in Verbindung mit den illustrierten Ausführungsbeispielen zu entnehmen.

In einer vorteilhaften Anwendung dient die lösungsgemäße Vorrichtung zum gasblasenfreien Befüllen eines pumpenbetriebenen, hohlleitungsgestützten Flüssigkeitskreislaufes mit einer Flüssigkeit, bei dem der hohlleitungsgestützte Flüssigkeitskreislauf an einer Stelle unterbrochen ist und zwei Fluidleitungsenden aufweist, von denen eine eine Zu- und die andere eine Ableitung des hohlleitungsgestützten Flüssigkeitskreislaufes darstellt. Zur fluiddichten Verbindung der lösungsgemäßen Beutelvorrichtung an den zu befüllenden pumpenbetriebenen , hohlleitungsgestützten Flüssigkeitskreislauf weist jeweils der erste und zweite Hohlkanalabschnitt ein außerhalb des Beutels befindliches Kanalende auf, die zum Konnektieren an die Zu- bzw. Ableitung des hohlleitungsgestützten Flüssigkeitskreislaufes ausgebildet sind. Auch in diesem Fall eignet sich zum fluiddichten Konnektieren beider Hohlleitungsabschnitte mit der Zu- und Ableitung des Flüssigkeitskreislaufes die Verwendung unterschiedlicher Materialpaarungen in Bezug auf ihre Elastizitätseigenschaften, so dass ein fluiddichtes Ineinanderfügen der Leitungen möglich wird. Selbstverständlich können auch jegliche weitere, dem Fachmann bekannte fluiddichte Flanschverbindungen vorgesehen werden, wie bspw. Luer-Lockverbindungen etc.

In besonders bevorzugter Weise dient die lösungsgemäße Vorrichtung dem Befüllen eines pumpenbetriebenen hohlleitungsgestützten Flüssigkeitskreislaufes, in dem ein Oxygenator zur Sauerstoffanreicherung von Blut eines Patienten integriert ist.

Unter Verwendung der vorstehend erläuterten Vorrichtung wird zudem ein Verfahren zum gasblasenfreien Befüllen eines pumpenbetriebenen hohlleitungsgestützten Flüssigkeitskreislaufes mit einer Flüssigkeit erläutert, bei dem der hohlleitungsgestützte Flüssigkeitskreislauf an einer Stelle unterbrochen ist und zwei Fluidleitungsenden aufweist, von denen eine, eine Zu- und eine andere eine Ableitung des hohlleitungsgestützten Flüssigkeitskreislaufes darstellen.

Das lösungsgemäße Verfahren zeichnet sich durch die Abfolge folgender Verfahrensschritt aus:
Zunächst gilt es den Beutel der vorstehend genannten lösungsgemäßen Art, bei dem das offene Kanalende des ersten Hohlkanalabschnittes innerhalb des Beutels mit dem Fluidleitungsabschnitt fluiddicht verbunden wird oder ist, bereitzustellen. Im Anschluss daran werden die Zuleitung mit dem ersten Hohlkanalabschnitt und die Ableitung mit dem zweiten Hohlkanalabschnitt des Beutels fluiddicht konnektiert. Anschließend erfolgt das Befüllen des hohlleitungsgestützten Flüssigkeitskreislaufes mit Flüssigkeit durch Einleiten der Flüssigkeit in das erste Filtervolumen, die nachfolgend durch die erste Filterwand in das angrenzende Beutelvolumen und von dort in die Ableitung des hohlleitungsgestützten Flüssigkeitskreislaufes gelangt, in dem pumpenbetrieben eine Förderrichtung der Flüssigkeit von der Ableitung zur Zuleitung eingeprägt wird. Hierdurch gelangt die Flüssigkeit über die Zuleitung, dem ersten Hohlkanalabschnitt und dem Fluidleitungsabschnitt in das erste oder falls vorhanden zweite Filtervolumen.

Der Befüllvorgang wird nach Erreichen einer vollständigen Befüllung des hohlleitungsgestützten Flüssigkeitskreislaufes sowie zumindest einer Teilbefüllung des Beutels beendet. Der pumpenbetriebene Flüssigkeitskreislauf wird auch nach Beenden des Befüllvorganges aufrechterhalten, bis die gesamte Flüssigkeitsmenge innerhalb des hohlleitungsgestützten Flüssigkeitskreislaufes sowie innerhalb des Beutels wenigstens einmal, vorzugsweise mehrfach die wenigstens erste Filterwand passiert hat.

Aufgrund der lichttransparenten Ausbildung der Beutelwand kann visuell überprüft werden, ob eine Gasblasenabscheidung innerhalb des Filters auftritt. Kommt sie sichtbar zum Erliegen, so ist dies ein erstes Indiz dafür, dass die innerhalb des hohlleitungsgestützten Flüssigkeitskreislaufes geführte Flüssigkeit vollständig gasblasenfrei ist. In diesem Fall wird der erste Hohlkanalabschnitt von dem Fluidleitungsabschnitt durch manuelles Abziehen gelöst und nachfolgend die innerhalb des Beutels befindlichen offenen Enden des ersten und zweiten Hohlkanalabschnittes miteinander fluiddicht verbunden. Das Lösen sowie das erneute Konnektieren erfolgen innerhalb des flüssigkeitsbefüllten Bereiches des Beutelvolumens, so dass sichergestellt ist, dass keinerlei Gaseinschlüsse während des Konnektierens des ersten und zweiten Hohlkanalabschnittes auftreten. Auch kann das Lösen und das erneute Konnektieren während des pumpenbetriebenen Flüssigkeitskreislaufes oder nach entsprechendem Stillstand des Flüssigkeitskreislaufes vorgenommen werden, je nach Situation und äußeren Umständen. Sämtliche Handgriffe zum Lösen und Konnektieren erfolgen aufgrund der flexiblen Beutelwand von außen über die Beutelwand.

Wird, wie vorstehend erläutert, zur Verbesserung der Filterwirkung innerhalb des ersten Filtereinsatzes ein zweiter Filtereinsatz vorgesehen, so mündet die Flüssigkeit, die über die Zuleitung des hohlleitungsgestützten Flüssigkeitskreislaufes dem Beutel zugeführt wird, zunächst innerhalb des zweiten Filtervolumens. Erst nach Durchtritt durch die zweite Filterwand und/oder durch eine entsprechende Öffnung innerhalb der zweiten Filterwand gelangt die Flüssigkeit in das erste Filtervolumen und nach Durchtritt durch die erste Filterwand in das übrige Beutelvolumen, aus dem die Flüssigkeit über den zweiten Hohlkanalabschnitt und die daran fluiddicht verbundene Ableitung in den hohlleitungsgestützten Flüssigkeitskreislauf zurückgeführt wird, in dem vorzugsweise ein Oxygenator integriert ist.

Nachdem der erste und zweite Hohlkanalabschnitt gasblasenfrei innerhalb des Beutels konnektiert worden sind, kann der Beutel entleert und bedarfsweise von den Hohlkanalabschnitten wenigstens teilweise entfernt werden. Hierzu sind an den fluiddicht ausgebildeten Verbindungsbereichen zwischen den ersten und zweiten Hohlkanalabschnitten und der Beutelwand geeignet auftrennbare Sollbruchstellen bzw. Fügebereiche vorgesehen.

Neben der vorstehend erläuterten schnellen und gasblasenfreien Befüllung eines pumpenbetriebenen hohlleitungsgestützten Flüssigkeitskreislaufes, in dem ein Oxygenator zur Sauerstoffanreicherung von Patientenblut integriert ist, kann die lösungsgemäß ausgebildete Beutelvorrichtung ebenfalls auch zur Befüllung beliebig geschlossener Flüssigkeitskreisläufe im Bereich von Wissenschaft und Technik eingesetzt werden. Hierzu sind lediglich die außerhalb des Beutels geführten Kanalenden des ersten und zweiten Hohlkanalabschnittes in einer an eine jeweils extern vorhandene Zu- und Ableitungsanschlussstruktur entsprechenden Art und Form auszubilden.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematische Darstellung der lösungsgemäßen Vorrichtung mit einem Beutel zur Befüllung eines pumpenbetriebenen hohlleitungsgestützten Flüssigkeitskreislaufes und
- Fig. 2: Darstellung eines Befüllvorganges eines extrakorporalen Kreislaufes mit Oxygenator

### Wege zur Ausführung der Erfindung, gewerbliche Verwendbarkeit

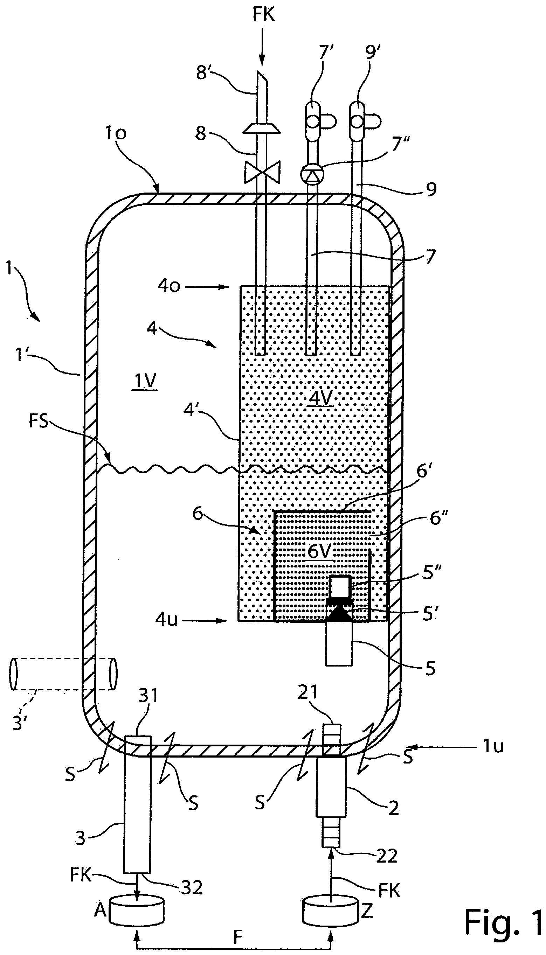

In Figur 1 ist in stark schematisierter Darstellung ein Beutel 1 illustriert, mit dem es möglich ist, einen pumpenbetriebenen, hohlleitungsgestützten Flüssigkeitskreislauf F blasenfrei mit einer Flüssigkeit FK zu befüllen. Hierzu sieht der Beutel 1 eine lichttransparente, elastisch ausgebildete Beutelwand 1' vor, die aus einem biokompatiblen elastisch verformbaren Kunststoffmaterial besteht. Die Beutelwand 1' umschließt zumindest teilweise ein Beutelvolumen 1V. Der Beutel 1 ist optional sack- oder tütenartig, d.h. an seiner oberen Beutelseite 1o offen, ausgebildet. Gleichwohl ist es möglich den Beutel 1 mit einer vollständig geschlossenen Beutelwand 1' auszuführen, so dass das innere Beutelvolumen 1V auch am oberen Beutelrand 1o fluiddicht abgeschlossen ist, wie dies in Figur 1 gezeigt ist.

An dem, dem oberen Beutelrand 1o diametral gegenüberliegenden unteren Beutelrand 1u sind zwei Hohlkanalabschnitte 2, 3 angebracht, die die Beutelwand 1' lokal fluiddicht durchragen, wobei beide Hohlkanalabschnitte 2, 3 jeweils ein innerhalb des Beutels 1 offen ausgebildetes Kanalende 21, 31 sowie ein außerhalb des Beutels 1 offen ausgebildetes Kanalende 22, 32 aufweisen. Beide Hohlkanalabschnitte 2, 3 schaffen jeweils einen freien Zugang zum inneren Beutelvolumen 1V.

Zusätzlich ist innerhalb des Beutels 1 ein erster Filtereinsatz 4 vorgesehen, dessen Filterwand 4' in Art eines Netzfilters ausgebildet ist, der vorzugsweise Filterporengrößen zwischen 20 und 200 µm, vorzugsweise 40, 80 oder 120 µm aufweist. Der erste Filtereinsatz ist wie der Beutel 1 selbst in einer ersten Ausführungsvariante beutel- bzw. tütenartig ausgebildet, d. h. die obere Seite 4o des ersten Filtereinsatzes 4 ist offen ausgestaltet, wohingegen die Filterwand 4' das erste innere Filtervolumen 4V ansonsten allseitig umfasst. In einer zweiten Ausführungsvariante ist es ebenso möglich den ersten Filtereinsatz 4 mit einer das erste Filtervolumen 4V vollständig umfassenden ersten Filterwand 4' auszubilden, wie dies in Figur 1 gezeigt ist.

Der erste Filtereinsatz 4 weist an seinem unteren Ende 4u einen die Filterwand 4' offen durchragenden Fluidleitungsabschnitt 5 auf, dessen Außenwand fluiddicht lokal an die erste Filterwand 4' angrenzt. Der Fluidleitungsabschnitt 5 ist vorzugsweise als kurzer Schlauchabschnitt ausgebildet, vorzugsweise bestehend aus einem elastischen Material, bspw. PVC. Längs des Fluidleitungsabschnittes 5 ist vorzugsweise ein Rückschlagventil 5' eingebracht, das verhindert, dass Flüssigkeit aus dem ersten Filtervolumen 4V, bzw. wie weiter unten erläutert, aus dem zweiten Filtervolumen 6V in das Beutelvolumen 1V gelangen kann. Zudem kann an dem im Filtervolumen 4V offen mündenden Ende des Fluidleitungsabschnittes 5 ein Strömungsdrosselelement 5" angebracht sein, das den Strömungsquerschnitt des Fluidleitungsabschnittes 5 endseitig reduziert, um so einen lokalen Druckanstieg längs des Fluidleitungsabschnittes 5 zu erzeugen, wodurch sich ein positiver Einfluss auf die Gasseparation einstellt.

Für ein lösbar fluiddichtes Konnektieren des Fluidleitungsabschnittes 5 mit dem innerhalb des Beutels befindlichen Kanalendes 21 des Hohlkanalabschnittes 2, der gegenüber dem Fluidleitungsabschnitt aus einem härteren Material gewählt ist, vorzugsweise Polycarbonat, sind die Durchmesserverhältnisse des Fluidleitungsabschnittes 5 sowie des Hohlkanalabschnittes 2 derart aufeinander abgestimmt, so dass der flexibel ausgestaltete Fluidleitungsabschnitt 5 fluiddicht über das Kanalende 21 des ersten Hohlkanalabschnittes 2 geschoben werden kann. Die hierfür erforderlichen Handgriffe erfolgen von außen über die lichttransparent und flexibel ausgebildete Beutelwand 1'. In gleicher Weise kann auch die vorstehend erläuterte Konnektierung durch geeignete Handgriffe von außen über die Beutelwand 1' wieder gelöst werden.

Der die Beutelwand 1' durchragende zweite Hohlkanalabschnitt 3 ist zu Zwecken eines fluiddichten Konnektierens mit dem innerhalb des Beutels 1 befindlichen Kanalendes 21 des ersten Hohlkanalabschnittes 2, wie der Fluidleitungsabschnitt 5 aus einem elastischen Material, vorzugsweise PVC ausgebildet, so dass das innerhalb des Beutels 1 befindliche offene Kanalende 31 des zweiten Hohlkanalabschnittes 3 über das offene Kanalende 21 des ersten Hohlkanalabschnittes 2 fluiddicht geschoben werden kann.

Selbstverständlich ist es möglich für ein lösbar fluiddichtes Konnektieren sowohl des Fluidleitungsabschnittes 5 mit dem ersten Hohlkanalabschnitt 2 sowie auch beider Hohlkanalabschnitte 2 und 3 geeignet ausgebildete alternative, fluiddichte Flanschverbindungsmechanismen, die dem Fachmann bekannt sind, bspw. Luer-Lockverbindungen, einzusetzen.

Im Falle des in Figur 1 illustrierten Ausführungsbeispiel sind der erste und zweite Hohlkanalabschnitt 2, 3 parallel zueinander orientiert und durchragen beabstandet zueinander die Filterwand 1' an der unteren Seite 1u des Beutels 1. Vorzugsweise bietet es sich an, den zweiten Hohlkanalabschnitt 3 dem ersten Hohlkanalabschnitt 2 zugewandt geneigt anzuordnen, um auf diese Weise das Konnektieren beider Hohlkanalabschnitte zu erleichtern. In diesem Zusammenhang böte es sich alternativ zum illustrierten Fall ebenso an, den zweiten Hohlkanalabschnitt 3 im Bereich der seitlichen Filterwand 1' mit einer Kanallängsachse, die orthogonal zur Kanallängsachse des zweiten Hohlkanalabschnittes 2 orientiert ist anzuordnen. Diese alternative Anordnung ist in Figur 1 durch den strichliert angedeuteten zweiten Hohlkanalabschnitt 3' dargestellt.

Ferner sieht eine bevorzugte Ausführungsform innerhalb des ersten Filtereinsatzes 4 einen zweiten Filtereinsatz 6 vor, dessen zweite Filterwand 6' ein zweites Filtervolumen 6V gemeinsam mit der unteren Seite 4u der ersten Filterwand 4' vollständig oder teilweise begrenzt, in den der Fluidleitungsabschnitt 5 mündet. Der zweite Filtereinsatz 6 ist gleichsam zum ersten Filtereinsatz 4 als Netzfilter ausgebildet und verfügt vorzugsweise über die gleiche Porengröße wie der erste Filtereinsatz. Das Vorsehen eines zweiten Filtereinsatzes 6 dient der sicheren Entgasung von gasbehafteten Fluidströmen, die die Beutelanordnung mit hohen Strömungsgeschwindigkeiten durchsetzt, die fünf Liter pro Minute und mehr, so bspw. sieben Liter pro Minute betragen.

Der zweite Filtereinsatz 6 weist vorzugsweise eine der Filterwand 4' abgewandte Öffnung 6" auf, durch die die Flüssigkeitsströmung nach entsprechender Strömungsablenkung in das erste Filtervolumen 4V gelangen kann. Neben einer ersten Entgasung dient in diesem Fall der zweite Filtereinsatz 6 auch der Strömungsablenkung, so dass die Flüssigkeitsströmung nicht mit großer Strömungsgeschwindigkeit auf die erste Filterwand 4' auftrifft.

Zum Zwecke der Befüllung des Beutels 1 mit Flüssigkeit ist eine Füllleitung 8 vorgesehen, die sowohl den offen oder geschlossen ausgebildeten oberen Beutelrand 1o sowie auch die obere Seite des ersten Filtereinsatzes 4o durchragt, so dass die über die Füllleitung 8 zugeführte Flüssigkeit zunächst in das erste Filtervolumen 4V gelangt. Vorzugsweise weist die Füllleitung 8 einen Anstechdorn 8' auf. Ferner mündet eine Entgasungsleitung 7 durch den oberen Beutelrand 1o über die obere Seite 4o des ersten Filtereinsatzes 4 in das erste Filtervolumen 4V, über die separierte Gasanteile entweichen bzw. abgesaugt werden können. In einer alternativen Ausbildungsform mündet die Entgasungsleitung 7 im oberen Bereich des Beutelvolumens 1V. Die Entgasungsleitung 7 weist vorzugsweise einen Dreiwegehahn 7' und ein Rückschlagventil 7" auf. Je nach Einsatzzweck können weitere Fluidleitungen 9 mit einem Dreiwegehahn in das erste Filtervolumen 4V oder das Beutelkvolumen 1V münden, die systembedingt von einem jeweils mit einer Flüssigkeit zu befüllenden, pumpengetriebenen hohlleitungsgestützten Flüssigkeitskreislauf F abhängen. Zweckmäßigerweise sind die Füllleitung 8 sowie die Entgasungsleitung 7 derart am Beutel 1 angeordnet, so dass sie über den oberen Beutelrand 1o in das Beutelvolumen 1V respektive in das Filtervolumen 4V hineinragen. Die weitere Fluidleitung 9 kann hingegen, je nach technischem Zweck, beliebig am Beutel 1 angeordnet sein, bspw. parallel zum ersten Hohlkanalabschnitt 2 verlaufen und offen in das Beutelvolumen 1V oder in das Filtervolumen 4V münden. Auch ist es denkbar, längs der weiteren Fluidleitung 9 zur Vermeidung eines Rückflusses ein Rückschlagventil vorzusehen.

Die vorstehend bezeichnete Beutelvorrichtung dient in erster Linie zur Befüllung eines hohlleitungsgestützten Flüssigkeitskreislaufes F für den repräsentativ in Figur 1 lediglich eine Ableitung A sowie Z illustriert ist, die mit den offenen Enden 22, 32 des ersten und zweiten Hohlkanalabschnittes 2, 3 fluiddicht konnektierbar sind.

Der Befüllvorgang des pumpenbetriebenen hohlleitungsgestützten Flüssigkeitskreislaufes F erfolgt in der Weise, dass zunächst der Fluidleitungsabschnitt 5 mit dem Ende 21 des ersten Hohlkanalabschnitt 2 fluiddicht verbunden wird. Im Weiteren erfolgt die Befüllung des Beutels 1 mit Flüssigkeit FK über die Füllleitung 8. Hierbei passiert die Flüssigkeit FK die erste Filterwand 4' und gelangt über den zweiten Hohlkanalabschnitt 3, der fluiddicht mit der Ableitung A verbunden ist, in den pumpenbetriebenen, hohlleitungsgestützten Flüssigkeitskreislauf F.

Die Befüllung des Beutels 1 mit Flüssigkeit FK erfolgt derart, so dass sich innerhalb des Beutels 1 ein Flüssigkeitsspiegel FS einstellt, der sich zumindest über dem Fluidleitungsabschnitt 5 befindet, vorzugsweise jedoch das gesamte zweite Filtervolumen 6V umfasst. Ein bevorzugter Flüssigkeitsspiegel FS ist in Figur 1 markiert, d. h. oberhalb des Flüssigkeitsspiegels FS herrschen normale atmosphärische Umgebungsbedingungen. Der Befüllvorgang wird beendet, sobald der gesamte Flüssigkeitskreislauf F mit Flüssigkeit gefüllt ist und sich der in Figur 1 illustrierte Flüssigkeitsspiegel FS innerhalb des Beutels 1 in etwa einstellt. Durch die wenigstens eine längs des Flüssigkeitskreislaufes F (nicht dargestellt) enthaltene Umlaufpumpe gelangt die Flüssigkeit aus dem Beutel 1 über die Ableitung A und der Zuleitung Z in das erste Filtervolumen 4V und im Falle des Vorhandenseins eines zweiten Filtereinsatzes 6 in das zweite Filtervolumen 6V. Jeweils bei Durchtritt der Flüssigkeit durch die Filterwand 6' sowie 4' erfolgt eine Gasabscheidung. Dies führt dazu, dass letztlich nach Durchtritt der Flüssigkeit FK durch die erste Filterwand 4' in das angrenzende Beutelvolumen 1V, die Flüssigkeit vollständig entgast, d.h. blasenfrei ist.

Der Vorgang der Entgasung der durch die Beutelanordnung und den daran fluiddicht angeschlossenen, pumpenbetriebenen, hohlleitungsgestützten Flüssigkeitskreislauf zirkulierenden Flüssigkeit wird beendet, nachdem keine weitere Gasphasenabscheidung innerhalb des ersten und zweiten Filtereinsatzes 4, 4 erfolgt. Dies kann im einfachsten Fall durch bloße Sichtkontrolle beurteilt werden.

Im Weiteren erfolgt ein Diskonnektieren bzw. Lösen des Fluidleitungsabschnittes 5 von dem ersten Hohlkanalabschnitt 2 und ein nachfolgendes fluiddichtes Verbinden des ersten und zweiten Hohlkanalabschnittes 3. Sowohl das Diskonnektieren sowie das Konnektieren erfolgen innerhalb des mit Flüssigkeit FK befüllten Beutelvolumens 1V, so dass jeglicher Gasblaseneinschluss während des Konnektiervorganges ausgeschlossen werden kann.

Nach fluiddichtem Konnektieren des ersten und zweiten Hohlkanalabschnittes 2, 3 wird der Beutel 1 entleert und von dem ersten und zweiten Hohlkanalabschnitt 2, 3 entfernt. Hierfür dienen innerhalb der Beutelwand 1' vorgesehene Sollbruchstellen S.

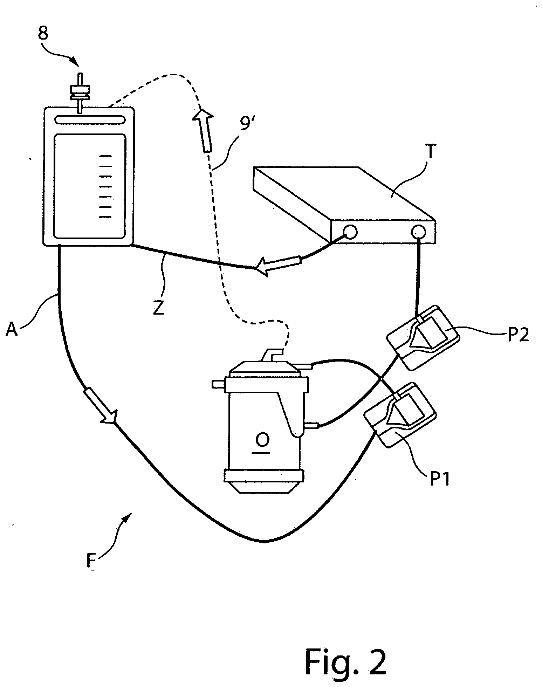

In Figur 2 ist ein bevorzugtes Anwendungsbeispiel der lösungsgemäßen Beutelvorrichtung illustriert. So dient die Beutelvorrichtung 1 dem Befüllen eines pumpenbetriebenen, hohlleitungsgestützten Flüssigkeitskreislaufes F, in dem ein Oxygenator O sowie ein als Tischlinie T bezeichneter Fluidleitungsabschnitt enthalten sind.

In einem ersten Schritt wird der Beutel 1 mit Flüssigkeit über die Füllleitung 8 befüllt. Hierbei ist die Zuleitung Z des pumpenbetriebenen hohlleitungsgestützten Flüssigkeitskreislaufes F abgeklemmt, um zu verhindern, dass Flüssigkeit entgegen der durch die Pumpen P1/P2 in den Flüssigkeitskreislauf eingeprägte Förderrichtung in den hohlleitungsgestützten Flüssigkeitskreislauf F gelangen kann. Somit gelangt die Flüssigkeit aus dem Beutel 1 über die Ableitung A Pumpengetrieben durch die Pumpe P1 in den Oxygenator O, der eine sog. Shunt-Leitung 9' aufweist, die zur Entlüftung des Oxygenators O dient und als Fluidleitung 9 in den Beutel mündet, wie vorstehend beschrieben. Nach entsprechender Befüllung des Oxygenators O wird die zweite Pumpe P2 in Betrieb genommen und die Abklemmung längs der Zuleitung Z gelöst. Somit gelangt die aus dem Oxygenator O austretende Flüssigkeit über den in der Tischlinie T zusammengefassten Fluidleitungsabschnitt und die Zuleitung Z in den Beutel 1. Hier erfolgt die vorstehend beschriebene Entgasung der Flüssigkeitsströmung mit Hilfe des wenigstens einen, vorzugsweise der zwei separat vorgesehenen Filtereinsätze. Nach erfolgreichem Beenden der Entgasung werden die Zu- und Ableitung A, Z durch Konnektieren der innerhalb des Beutels 1 einmündenden ersten und zweiten Hohlkanalabschnitte gasblasenfrei und fluiddicht verbunden.

Der Befüllvorgang erfolgt aufgrund des Pumpenbetriebes P1/P2 innerhalb kürzester Zeit und kann zudem gasblasenfrei sichergestellt werden.

### Bezugszeichenliste

- 1: Beutelförmiges Behältnis, Beutel
- 1V: Beutelvolumen
- 1': Beutelwand
- 1o: Oberer Beutelrand
- 1u: Unterer Beutelrand
- 2: Erster Hohlkanalabschnitt
- 3: Zweiter Hohlkanalabschnitt
- 4: Erster Filtereinsatz
- 4V: Erstes Filtervolumen
- 4': Erste Filterwand
- 4o: Oberer Filterrand
- 4u: Unterer Filterrand
- 5: Fluidleitungsabschnitt
- 5': Rückschlagventil
- 5": Strömungsdrosselelement
- 6: Zweiter Filtereinsatz
- 6': Zweites Filtervolumen
- 6V: Zweite Filterwand
- 6": Öffnung
- 7: Entgasungsleitung
- 7': Dreiwegehahn
- 7": Rückschlagventil
- 8: Füllleitung
- 9: Fluidleitung
- 21: Offenes Ende des ersten Hohlkanalabschnitts innerhalb des Beutels
- 22: Offenes Ende des ersten Hohlkanalabschnitts außerhalb des Beutels
- 31: Offenes Ende des zweiten Hohlkanalabschnitts innerhalb des Beutels
- 32: Offenes Ende des zweiten Hohlkanalabschnitts außerhalb des Beutels
- F: Flüssigkeitskreislauf
- FK: Flüssigkeit
- FS: Flüssigkeitsspiegel
- A: Ableitung
- S: Sollbruchstelle
- Z: Zuleitung
- P1,P2: Pumpe
- O: Oxygenator
- T: Tischlinie

## Patentansprüche

1. Vorrichtung mit einem beutelförmigen Behältnis (1), im weiteren Beutel genannt, dessen Beutelwand (1') zumindest in einem Bereich flexibel ausgebildet ist, in dem zwei Hohlkanalabschnitte (2, 3), ein erster (2) und ein zweiter (3) Hohlkanalabschnitt, die Beutelwand (1') fluiddicht durchragen, die jeweils ein offenes Kanalende innerhalb (21, 31) des Beutels (1) aufweisen, die zum lösbaren fluiddichten Konnektieren miteinander ausgebildet sind,
**dadurch gekennzeichnet, dass** innerhalb des Beutels (1) ein erster Filtereinsatz (4) eingebracht ist, der innerhalb eines von dem Beutel (1) zumindest teilweise umfassten Volumens (1V), dem sogenannten Beutelvolumen, ein erstes inneres Filtervolumen (4V) mit einer ersten Filterwand (4') zumindest teilweise umfasst, durch die ein mit der ersten Filterwand (4') verbundener, schlauchförmiger Fluidleitungsabschnitt (5) hindurchragt, der beidseitig offen ausgebildet ist und sowohl mittel- oder unmittelbar in das erste Filtervolumen (4V) als auch unmittelbar in das Beutelvolumen (1V) mündet, und
dass das innerhalb des Beutels (1) befindliche Kanalende (21) des ersten Hohlkanalabschnittes (2) zum lösbar fluiddichten Konnektieren mit dem offen in das Beutelvolumen (1V) mündenden Fluidleitungsabschnitt (5) ausgebildet ist.

2. Vorrichtung nach Anspruch 1,
**dadurch gekennzeichnet, dass** innerhalb des ersten Filtervolumens (4V) ein zweiter Filtereinsatz (6) eingebracht ist, der ein zweites Filtervolumen (6V) mit einer zweiten Filterwand (6') zumindest teilweise umschließt, in das der in das erste Filtervolumen (4V) mündende Fluidleitungsabschnitt (5), mündet, und dass die zweite Filterwand (6') fluiddicht an dem Fluidleitungsabschnitt (5) angrenzt.

3. Vorrichtung nach Anspruch 1 oder 2,
**dadurch gekennzeichnet, dass** der erste und zweite Hohlkanalabschnitt (2, 3) jeweils ein Kanalende (22, 32) außerhalb des Beutels (1) aufweisen, die zum fluiddichten Konnektieren an eine Zu- (Z) und Ableitung (A) eines hohlleitungsgestützten Flüssigkeitskreislaufes (F) ausgebildet sind.

4. Vorrichtung nach einem der Ansprüche 1 bis 3,
**dadurch gekennzeichnet, dass** wenigstens eine Entgasungsleitung (7) in das erste Filtervolumen (4V) mündet.

5. Vorrichtung nach einem der Ansprüche 1 bis 4,
**dadurch gekennzeichnet, dass** wenigstens eine Füllleitung (8) in das erste Filtervolumen (4V) mündet, über die der Beutel (1) mit der Flüssigkeit befüllbar ist.

6. Vorrichtung nach einem der Ansprüche 1 bis 5,
**dadurch gekennzeichnet, dass** wenigstens eine Fluidleitung (9) in das erste Filtervolumen (4V) mündet.

7. Vorrichtung nach den Ansprüchen 4 bis 6,
**dadurch gekennzeichnet, dass** zumindest die Entgasungsleitung (7) und die Füllleitung (8) dem Fluidleitungsabschnitt (5) diametral gegenüberliegend in das erste Filtervolumen (4') münden.

8. Vorrichtung nach Anspruch 7,
dass der Beutel (1) zu Seiten der Entgasungsleitung (7) und Füllleitung (8) offen ausgebildet ist, oder
dass der Beutel zu Seiten der Entgasungsleitung (7) und Füllleitung (8) geschlossen ausgebildet, wobei die Entgasungsleitung (7) und Füllleitung (8) die Beutelwand (1') fluiddicht durchdringen.

9. Verwendung der Vorrichtung nach einem der Ansprüche 3 bis 8 zum gasblasenfreien Befüllen eines pumpenbetriebenen, hohlleitungsgestützten Flüssigkeitskreislaufes (F) mit einer Flüssigkeit, bei dem der hohlleitungsgestützte Flüssigkeitskreislauf an einer Stelle unterbrochen ist und zwei Fluidleitungsenden aufweist, von denen eine die Zu- (Z) und eine andere die Ableitung (A) des hohlleitungsgestützten Flüssigkeitskreislaufes (F) darstellen.

10. Verwendung nach Anspruch 9,
**dadurch gekennzeichnet, dass** in dem hohlleitungsgestützten Flüssigkeitskreislauf (F) ein Oxygenator zur Sauerstoffanreicherung von Blut eines Patienten integriert ist.

11. Verfahren zum gasblasenfreien Befüllen eines pumpenbetriebenen, hohlleitungsgestützten Flüssigkeitskreislaufes (F) mit einer Flüssigkeit, bei dem der hohlleitungsgestützte Flüssigkeitskreislauf (F) an einer Stelle unterbrochen ist und zwei Fluidleitungsenden aufweist, von denen eine eine Zu- (Z) und eine andere eine Ableitung (A) des hohlleitungsgestützten Flüssigkeitskreislaufes (F) darstellen, **gekennzeichnet durch** folgende Verfahrensschritte:
- Bereitstellen eines Beutels (1) nach einem der Ansprüche 3 bis 8, bei dem das offene Kanalende (21) des ersten Hohlkanalabschnittes (2) innerhalb des Beutels (1) mit dem Fluidleitungsabschnitt (5) fluiddicht verbunden wird oder verbunden ist,
- Fluiddichtes Konnektieren der Zuleitung (Z) mit dem ersten Hohlkanalabschnitt (2) sowie der Ableitung (A) mit dem zweiten Hohlkanalabschnitt (3),
- Befüllen des hohlleitungsgestützten Flüssigkeitskreislaufes (F) mit der Flüssigkeit **durch** Einleiten der Flüssigkeit in das erste Filtervolumen (4V), die nachfolgend **durch** die erste Filterwand (4') in das angrenzende Beutelvolumen (1V) und von dort in die Ableitung (A) des hohlleitungsgestützten Flüssigkeitskreislaufes (F) gelangt, in dem pumpenbetrieben eine Förderrichtung der Flüssigkeit von der Ableitung (A) zur Zuleitung (Z) eingeprägt wird, wodurch die Flüssigkeit über die Zuleitung (Z), dem ersten Hohlkanalabschnitt (2) und dem Fluidleitungsabschnitt (5) in das erste (4V) oder zweite (6V) Filtervolumen gelangt,
- Beenden des Befüllens nach Erreichen einer vollständigen Befüllung des hohlleitungsgestützten Flüssigkeitskreislaufes (F) sowie einer Befüllung des Beutels (1) derart, dass das erste Filtervolumen (4V) und das angrenzende Beutelvolumen (1V) jeweils teilweise befüllt sind,
- Aufrechterhalten des pumpenbetriebenen Flüssigkeitskreislaufes (F) bis die gesamte Flüssigkeit innerhalb des hohlleitungsgestützten Flüssigkeitskreislaufes (F) sowie innerhalb des Beutels (1) wenigstens einmal die erste Filterwand (4') passiert sowie
- Lösen des konnektierten ersten Hohlkanalabschnittes (2) von dem Fluidleitungsabschnitt (5) und fluiddichtes Konnektieren des ersten (2) und zweiten (3) Hohlkanalabschnittes innerhalb des Beutels (1) während des pumpenbetriebenen Flüssigkeitskreislaufes (F) oder nach Stillstand des Flüssigkeitskreislaufes (F), wobei das Lösen und Konnektieren innerhalb des mit Flüssigkeit befüllten Beutelvolumens (1V) erfolgt.

12. Verfahren nach Anspruch 11,
**dadurch gekennzeichnet, dass** ein Beutel gemäß Anspruch 2 bereitgestellt wird, und
dass die Flüssigkeit aus dem Fluidleitungsabschnitt (5) die zweite Filterwand (6') und anschließend die erste Filterwand (4') passiert.

13. Verfahren nach Anspruch 11 oder 12,
**dadurch gekennzeichnet, dass** zumindest ein in dem hohlleitungsgestützten, pumpenbetriebenen Flüssigkeitskreislauf integrierter Oxygenator befüllt wird.

14. Verfahren nach einem der Ansprüche 11 bis 13,
**dadurch gekennzeichnet, dass** nach dem Konnektieren des ersten (2) und zweiten (3) Hohlkanalabschnittes innerhalb des Beutels (1) der Beutel entleert und/oder von den Hohlkanalabschnitten (2,3) zumindest teilweise entfernt wird.

15. Verfahren nach einem der Ansprüche 11 bis 14,
**dadurch gekennzeichnet, dass** die gesamte in dem hohlleitungsgestützten Flüssigkeitskreislauf (F) sowie dem Beutel enthaltene Flüssigkeitsmenge durch wenigstens die erste Filterwand (4') gefördert wird, bis sich ein durch wenigstens die erste Filtereinheit vorgegebene Filterwirkung einstellender, minimaler Gasblasengehalt in der Flüssigkeit erreicht wird.

## Claims

1. A device with a bag-shaped container (1), which is simply referred to as bag hereafter and the bag wall (1') of which is designed flexible at least in a region, in which two hollow channel sections (2, 3), namely a first (2) and a second (3) hollow channel section, pass through the bag wall (1') in a fluid-tight manner, wherein said hollow channel sections respectively have an open channel end (21, 31) that is located within the bag (1) and designed for connecting the open channel ends to one another in a separable and fluid-tight manner,
**characterized in that** a first filter insert (4) is introduced within the bag (1) and at least partially encompasses a first inner filter volume (4V) with a first filter wall (4') within a volume (1V) that is at least partially encompassed by the bag (1), namely the so-called bag volume, wherein a tubular fluid line section (5) passes through the first filter wall (4') and is connected thereto,
and wherein said fluid line section is open on both sides and indirectly or directly leads into the first filter volume (4V), as well as directly into the bag volume (1V), and **in**
**that** the channel end (21) of the first hollow channel section (2), which is located within the bag (1), is designed for being connected to the open fluid line section (5) leading into the bag volume (1V) in a separable and fluid-tight manner.

2. The device according to claim 1,
**characterized in that** a second filter insert (6) is introduced within the first filter volume (4V) and at least partially encloses a second filter volume (6V) with a second filter wall (6'), into which the fluid line section (5) leading into the first filter volume (4V) leads, and **in that** the second filter wall (6') borders on the fluid line section (5) in a fluid-tight manner.

3. The device according to claim 1 or 2,
**characterized in that** the first and second hollow channel sections (2, 3) respectively have a channel end (22, 32) that is located outside the bag (1) and designed for being respectively connected to a supply line (Z) and a discharge line (A) of a hollow line-supported liquid circuit (F) in a fluid-tight manner.

4. The device according to one of claims 1 to 3,
**characterized in that** at least one degassing line (7) leads into the first filter volume (4V).

5. The device according to one of claims 1 to 4,
**characterised in that** at least one filling line (8) for filling the bag (1) with the liquid leads into the first filter volume (4V).

6. The device according to one of claims 1 to 5,
**characterized in that** at least one fluid line (9) leads into the first filter volume (4V).

7. The device according to claims 4 to 6,
**characterised in that** at least the degassing line (7) and the filling line (8) lead into the first filter volume (4') diametrically opposite of the fluid line section (5).

8. The device according to claim 7,
**characterised in that** the bag (1) is designed open on the side of the degassing line (7) and the filling line (8) or in
that the bag is designed closed on the side of the degassing line (7) and the filling line (8), wherein the degassing line (7) and the filling line (8) pass through the bag wall (1') in a fluid-tight manner.

9. A utilization of the device according to one of claims 3 to 8 for filling a pump-operated, hollow line-supported liquid circuit (F) with a liquid without forming gas bubbles, wherein the hollow line-suppdrted liquid circuit is interrupted at one point and has two fluid line ends, one of which represents a supply line (z) and the other one of which represents a discharge line (A) of the hollow line-supported liquid circuit (F).

10. The utilization according to claim 9,
**characterized in that** an oxygenator is integrated into the hollow line-supported liquid circuit (F) in order to oxygenate the blood of a patient.

11. A method for filling a pump-operated, hollow line-supported liquid circuit (F) with liquid without forming gas bubbles, wherein the hollow line-supported liquid circuit (F) is interrupted at one point and has two fluid line ends, one of which represents a supply line (Z) and the other one of which represents a discharge line (A) of the hollow line-supported liquid circuit (F), **characterized by** the following steps:
- supplying a bag (1) according to one of claims 3 to 11, in which the open channel end (21) of the first hollow channel section (2) can be or is connected to the fluid line section (5) in a fluid-tight manner within the bag (1),
- connecting the supply line (z) to the first hollow channel section (2) in a fluid-tight manner and connecting the discharge line (A) to the second hollow channel section (3) in a fluid-tight manner,
- filling the hollow line-supported liquid circuit (F) with liquid by introducing the liquid into the first filter volume (4V), wherein said liquid subsequently flows into the adjacent bag volume (1V) through the first filter wall (4') and from there into the discharge line (A) of the hollow line-supported liquid circuit (F), in which the liquid is transported in the direction from the discharge line (A) to the supply line (Z) in a pump-operated manner such that the liquid flows into the first (4V) or the second (6V) filter volume through the supply line (Z), the first hollow channel section (2) and the fluid line section (5),
- completing the filling process after the hollow line-supported liquid circuit (F) has been completely filled and the bag (1) has been filled to such a degree that the first filter volume (4V) and the adjacent bag volume (1V) respectively are partially filled,
- sustaining the pump-operated liquid circuit (F) until the entire liquid within the hollow line-supported liquid circuit (F) and within the bag (1) has passed through the first filter wall (4') at least once, and
- separating the connected first hollow channel section (2) from the fluid line section (5) and producing a fluid-tight connection between the first (2) and the second (3) hollow channel section within the bag (1) during the operation of the liquid circuit (F) by means of the pump or after a standstill of the liquid circuit (F), wherein this separation and connection take place within the bag volume (1V) filled with liquid.

12. The method according to claim 11,
**characterized in that** a bag according to claim 2 is supplied, and **in**
**that** the liquid from the fluid line section (5) flows through the second filter wall (6') and subsequently through the first filter wall (4').

13. The method according to claim 11 or 12,
**characterized in that** at least one oxygenator integrated into the hollow line-supported, pump-operated liquid circuit is filled.

14. The method according to one of claims 11 to 13,
**characterized in that** the bag is emptied and/or at least partially removed from the hollow channel sections (2, 3) after connecting the first (2) and the second (3) hollow channel sections within the bag (1).

15. The method according to one of claims 11 to 14,
**characterized in that** the entire amount of liquid contained in the hollow line-supported liquid circuit (F) and the bag is conveyed through at least the first filter wall (4') until a minimal gas bubble content in the liquid is reached due to the filter effect of at least the first filter unit.

## Revendications

1. Dispositif avec un conteneur en forme de sac (1), dénommé ci-après sac, dont la paroi de sac (1') est constituée souple au moins dans une zone dans laquelle deux sections à conduit creux (2, 3) une première (2) et une deuxième (3) section à conduit creux, traversent de manière étanche au fluide la paroi de sac (1'), qui comportent respectivement une extrémité de conduit ouverte à l'intérieur (21, 31) du sac (1), qui sont constituées pour la connexion amovible étanche au fluide l'une avec l'autre,
**caractérisé en ce qu'**à l'intérieur du sac (1) un premier élément de filtre (4) est monté, qui comprend à l'intérieur d'un volume (1V) entouré au moins en partie par le sac (1), ledit volume de sac, au moins en partie un premier volume de filtre intérieur (4V) avec une première paroi de filtre (4') à travers laquelle passe une section de conduit de fluide (5) en forme de flexible, reliée à la première paroi de filtre (4'), qui est constituée ouverte des deux côtés et débouche tant directement ou indirectement dans le premier volume de filtre (4V) que directement dans le volume de sac (1V), et
**en ce que** l'extrémité de conduit (21) se trouvant à l'intérieur du sac (1) de la première section de conduit creux (2) est constituée pour la connexion étanche au fluide amovible avec la section de conduit de fluide (5) débouchant ouverte dans le volume de sac (1V) .

2. Dispositif selon la revendication 1,
**caractérisé en ce qu'**à l'intérieur du premier volume de filtre (4V) est monté un deuxième élément de filtre (6) qui entoure au moins en partie un deuxième volume de filtre (6V) avec une deuxième paroi de filtre (6') dans lequel débouche la section de conduit de fluide (5) débouchant dans le premier volume de filtre (4V) et **en ce que** la deuxième paroi de filtre (6') est contigüe de manière étanche au fluide à la section de conduit de fluide (5).

3. Dispositif selon la revendication 1 ou 2,
**caractérisé en ce que** la première et la deuxième section de conduit creux (2, 3) comportent respectivement une extrémité de conduit (22, 32) en dehors du sac (1), qui sont constituées pour la connexion étanche au fluide à un conduit d'arrivée (Z) et un conduit de départ (A) d'un circuit de liquide assisté par un conduit creux (F).

4. Dispositif selon l'une quelconque des revendications 1 à 3,
**caractérisé en ce qu'**au moins un conduit de dégazage (7) débouche dans le premier volume de filtre (4V).

5. Dispositif selon l'une quelconque des revendications 1 à 4,
**caractérisé en ce qu'**au moins un conduit de remplissage (8) débouche dans le premier volume de filtre (4V) par le biais duquel le sac (1) peut être rempli avec le liquide.

6. Dispositif selon l'une quelconque des revendications 1 à 5,
**caractérisé en ce qu'**au moins un conduit de fluide (9) débouche dans le premier volume de filtre (4V).

7. Dispositif selon les revendications 4 à 6,
**caractérisé en ce qu'**au moins le conduit de dégazage (7) et le conduit de remplissage (8) débouchent dans le premier volume de filtre (4') diamétralement opposé à la section de conduit de fluide (5).

8. Dispositif selon la revendication 7,
**caractérisé en ce que** le sac (1) est conçu ouvert vers les côtés du conduit de dégazage (7) et du conduit de remplissage (8), ou
**en ce que** le sac est conçu fermé vers les côtés du conduit de dégazage (7) et du conduit de remplissage (8), le conduit de dégazage (7) et le conduit de remplissage (8) traversant la paroi de sac (1') de manière étanche au fluide.

9. Utilisation du dispositif selon l'une quelconque des revendications 3 à 8 pour le remplissage sans bulles de gaz d'un circuit de liquide actionné par pompe, assisté par un conduit creux (F) avec un liquide, pour lequel le circuit de liquide assisté par un conduit creux est interrompu en un endroit et comporte deux extrémités de conduit de fluide dont l'un représente le conduit d'arrivée (Z) et l'autre le conduit de départ (A) du circuit de liquide assisté par un conduit creux (F).

10. Utilisation selon la revendication 9,
**caractérisé en ce qu'**un oxygénateur pour l'enrichissement en oxygène du sang d'un patient est intégré dans le circuit de liquide assisté par un conduit creux (F).

11. Procédé pour le remplissage sans bulles de gaz d'un circuit de liquide (F) actionné par une pompe assisté par un conduit creux avec un liquide, pour lequel le circuit de liquide assisté par un conduit creux (F) est interrompu à un endroit et comporte deux extrémités de conduit de fluide, dont l'un représente le conduit d'arrivée (Z) et l'autre le conduit de départ (A) du circuit de liquide assisté par un conduit creux (F), **caractérisé par** les étapes de procédé suivantes :
- préparation d'un sac (1) selon l'une quelconque des revendications 3 à 8, pour lequel l'extrémité de conduit ouverte (21) de la première section de conduit creux (2) à l'intérieur du sac (1) sera ou est reliée de manière étanche au fluide à la section de conduit de fluide (5),
- connexion étanche au fluide du conduit d'arrivée (Z) à la première section de conduit creux (2) ainsi que du conduit de départ (A) à la deuxième section de conduit creux (3),
- remplissage du circuit de liquide assisté par un conduit creux (F) avec le liquide par introduction du liquide dans le premier volume de filtre (4V), qui parvient ensuite par la première paroi de filtre (4') dans le volume de sac limitrophe (1V) et de là dans le conduit de départ (A) du circuit de liquide assisté par un conduit creux (F), dans lequel une direction de transport du liquide est imprimée sous l'action d'une pompe, du conduit de départ (A) au conduit d'arrivée (Z), le liquide parvenant par le biais du conduit d'arrivée (Z), de la première section de conduit creux (2) et de la section de conduit de fluide (5) dans le premier (4V) ou le deuxième volume de filtre (6V),
- finition du remplissage après avoir atteint un remplissage complet du circuit de liquide assisté par un conduit creux (F) ainsi qu'un remplissage du sac (1) de telle sorte que le premier volume de filtre (4V) et le volume de sac limitrophe (1V) sont respectivement en partie remplis,
- maintien du circuit de liquide assisté par un conduit creux (F) jusqu'à ce que tout le liquide à l'intérieur du circuit de liquide assisté par un conduit creux (F) ainsi qu'à l'intérieur du sac (1) passe au moins une fois la première paroi de filtre (4'), ainsi que
- séparation de la première section de conduit creux connectée (2) de la section de conduit de fluide (5) et connexion étanche au fluide de la première (2) et de la deuxième (3) section de conduit creux à l'intérieur du sac (1) pendant le circuit de liquide actionné par une pompe (F) ou après arrêt du circuit de liquide (F), la séparation et la connexion ayant lieu à l'intérieur du volume du sac (1V) rempli de liquide.

12. Procédé selon la revendication 11,
**caractérisé en ce qu'**un sac est préparé selon la revendication 2, et
**en ce que** le liquide de la section du conduit de fluide (5) passe la deuxième paroi de filtre (6') et ensuite la première paroi de filtre (4').

13. Procédé selon la revendication 11 ou 12,
**caractérisé en ce qu'**au moins un oxygénateur intégré dans le circuit de liquide actionné par une pompe, assisté par un conduit creux, est rempli.

14. Procédé selon l'une quelconque des revendications 11 à 13,
**caractérisé en ce qu'**après la connexion de la première (2) et de la deuxième (3) section de conduit creux à l'intérieur du sac (1), le sac est vidé et/ou est au moins en partie enlevé des sections de conduit creux (2, 3).

15. Procédé selon l'une quelconque des revendications 11 à 14,
**caractérisé en ce que** toute la quantité de liquide contenue dans le circuit de liquide assisté par un conduit creux (F) ainsi que dans le sac est transportée à travers au moins la première paroi de filtre (4') jusqu'à ce qu'une teneur minimale en bulles de gaz s'établissant par effet de filtrage prédéfini par au moins la première unité de filtre, soit atteinte dans le liquide.
